(19) Europäisches Patentamt / European Patent Office / Office européen des brevets

(11) **EP 2 808 025 B1**

(12) **EUROPEAN PATENT SPECIFICATION**

(45) Date of publication and mention
of the grant of the patent:
**13.07.2016 Bulletin 2016/28**

(21) Application number: **13740840.7**

(22) Date of filing: **22.01.2013**

(51) Int Cl.:
*A61K 31/7004* (2006.01)   *A61K 31/573* (2006.01)
*A61K 31/56* (2006.01)   *A61K 8/60* (2006.01)
*A61K 8/63* (2006.01)   *A61P 1/04* (2006.01)
*A61P 11/06* (2006.01)   *A61P 17/00* (2006.01)
*A61P 17/02* (2006.01)   *A61P 17/10* (2006.01)
*A61P 37/02* (2006.01)   *A61P 37/08* (2006.01)
*A61P 43/00* (2006.01)   *A61Q 19/00* (2006.01)
*A61Q 19/08* (2006.01)   *A61K 9/00* (2006.01)

(86) International application number:
**PCT/JP2013/051159**

(87) International publication number:
**WO 2013/111729 (01.08.2013 Gazette 2013/31)**

(54) **PHARMACEUTICAL COMPOSITION AND QUASI-DRUG COSMETIC USING SAME**

PHARMAZEUTISCHE ZUSAMMENSETZUNG UND QUASI-ARZNEIMITTEL-KOSMETIKUM DAMIT

COMPOSITION PHARMACEUTIQUE ET PRODUIT COSMÉTIQUE QUASI-MÉDICAMENTEUX UTILISANT CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.01.2012 JP 2012011342**

(43) Date of publication of application:
**03.12.2014 Bulletin 2014/49**

(73) Proprietor: **CAC Corporation**
**Nagareyama-shi,**
**Chiba 270-0114 (JP)**

(72) Inventors:
• **YAMADA, Hajime**
**Nagareyama-shi**
**Chiba 270-0114 (JP)**
• **YAMADA, Akira**
**Nagareyama-shi**
**Chiba 270-0114 (JP)**
• **YAMADA, Jiro**
**Nagareyama-shi**
**Chiba 270-0114 (JP)**

• **YAMADA, Kiyoko**
**Nagareyama-shi**
**Chiba 270-0114 (JP)**
• **MATSUSHITA, Kenji**
**Nagareyama-shi**
**Chiba 270-0114 (JP)**

(74) Representative: **Potter Clarkson LLP**
**The Belgrave Centre**
**Talbot Street**
**Nottingham NG1 5GG (GB)**

(56) References cited:
WO-A1-2006/121156   CN-A- 1 562 357
JP-A- 2006 045 186   US-A- 5 145 679
US-A- 5 885 978   US-A1- 2005 175 640

• DATABASE WPI Week 199741 Thomson Scientific, London, GB; AN 1997-436149 XP002732197, & CN 1 116 095 A (ANSHAN IRON & STEEL CO) 7 February 1996 (1996-02-07)

## Description

### TECHNICAL FIELD

The present invention relates to a composition which comprises glucose and a corticosteroid for use in promoting tissue repair in a subject in need thereof, wherein the corticosteroid is dexamethasone or dexamethasone sodium phosphate, or at least one selected from hydrocortisone, prednisolone, dexamethasone and betamethasone, and the molar ratio of glucose / corticosteroid is 10 to $10^2$.

This application claims priority on Japanese Patent Application No. 2012-11342 filed on January 23, 2012.

### BACKGROUND ART

Pharmaceutical compositions such as a wound treating agent and an anti-inflammatory agent have been used as a treatment for cell damage (endogenous damage) caused by internal factors such as mental stress and poor blood circulation or a treatment for cell damage (exogenous damage) caused by external factors such as a viral or bacterial infection, physical damage, and the like.

As a pharmaceutical composition which can be used for treating cell damage, pharmaceutical compositions have been proposed which include polysaccharides and/or oligosaccharide which have a repairing action with respect to bio-tissue (hereinafter, it may be simply referred to as "tissue") For example, a repairing agent and a prevention agent for treating cell damage or tissue disorder have been proposed, wherein polysaccharides and/or oligosaccharide thereof are mixed and/or blended therein, and the polysaccharides and/or oligosaccharide include one or more kinds of a monosaccharide which constitutes a sugar chain of glycoprotein and a sugar chain of glycolipid as a constituent and have physiological activity with respect to mammals. (For example, refer to Patent document 1.)

On the other hand, it has been reported that, although glucose is added in an injection for the purpose of feeding sugar at the time of low blood sugar or circulation collapse, treating hyperkalemia, or feeding water or supplying energy parenterally, the glucose delays tissue repair. (For example, refer to Non-Patent document 1.)

Furthermore, as a pharmaceutical composition which is used for treating cell damage, there is a pharmaceutical composition which includes a steroid. A steroid is a compound which has a steroid skeleton and is widely used for pharmacotherapy as a material which has strong anti-inflammatory action, immunosuppressive action and the like.

A steroid is a compound which is originally generated from an internal organ of a living body and generally means corticosteroids. A steroid has a wide application range, and has been known as one of drugs which have the largest number of indications. For example, as diseases to which a steroid is applicable, bronchial asthma, atopic dermatitis, hay fever, allergic rhinitis, sudden deafness, rheumatoid arthritis, collagen disease, nephrotic syndrome, ulcerative colitis and the like can be cited.

Inflammation is induced by a material such as prostaglandin, leukotriene and the like which function as a mediator. A steroid is incorporated into a cell and activates a gene to synthesize protein lipocortin, and the protein lipocortin inhibits phospholipase A2. As a result, the synthesis of prostaglandin and leukotriene is suppressed, and therefore an anti-inflammatory action is exhibited.

In addition, it is considered that a steroid inhibits the expression of an inflammatory cytokine gene and cyclooxygenase-2 (COC-2) gene, which is relevant to biosynthesis of prostaglandin.

The above functions are considered as a main action mechanism of a steroid with respect to the anti-inflammatory action thereof.

When a steroid is used for a long period of time or used frequently, a sufficient amount of the steroid exists in a living body. There is a problem that, production of the steroid in the living body is reduced if the steroid amount in a living body increases. In addition, there is a problem that the steroid tends to inhibit normal cell growth (proliferation inhibiting action) and to delay the repair of cell tissue.

With respect to the above problems, an anti-inflammatory agent in which a steroid and scutellaria root are used in combination has been proposed. (For example, refer to

Patent Document 2.) According to the invention of Patent document 2, it is possible to suppress apoptosis action of a steroid with respect to normal cells, while maintaining the anti-inflammatory action of a steroid.

### Conventional art documents

### Patent documents

Patent Document 1: Japanese Unexamined Patent Application, First Publication No. 2008-273919

Patent Document 2: Japanese Unexamined Patent Application, First Publication No. 2007-182384

Patent Document 3: US Patent No. 5,885,978 describes an external therapeutic composition for dermatitis comprising an aqueous solution including a therapeutically effective amount of an adrenal cortical steroid, a cyclodextrin, polysaccharides, and a carrier such as water, prepared by clathrating the adrenal cortical steroid in the cyclodextrin using a homomixer to form a clathrate, and adding the clathrate to an aqueous solution of polysaccharides, while being stirred uniformly, to dissolve the clathrate in the aqueous solution, as well as a method for the treatment of dermatitis in a mammalian subject, which comprises administering to said subject a therapeutically effective amount of the external therapeutic composition for dermatitis described above.

Patent Document 4: US Patent Application, Publication No. US2005/0175640 describes an external medicine for treating dermatitis comprising 0.025 to 0.5% by weight of the adrenocortical steroid, 0.2 to 30% by weight of the cyclodextrin, 0.5 to 55% by weight of dextran or pullulan dissolved in an aqueous solution containing polysaccharide; and 0.5 to 55% by weight of xyloglucan, trehalose, laminaran, krestin, and pectin. As discussed in US2005/0175640, grape sugar, mutan, lentinan, sodium chloride, and potassium chloride are comprised in the medicine.

Non Patent document 1: Spravchikov N et al. "GLUCOSE EFFECTS ON SKIN KERATINOCYTES IMPLICATIONS FOR DIABETES SKIN COMPLICATIONS", Diabetes 50, p. 1627 to 1635, 2001

However, a pharmaceutical composition which can further promote tissue repair, or can more effectively prevent cell damage is required.

DISCLOSURE OF INVENTION

Problem to be solved by the Invention

Accordingly, the present invention provides a composition which comprises glucose and a corticosteroid for use in promoting tissue repair in a subject in need thereof, wherein the corticosteroid is dexamethasone or dexamethasone sodium phosphate, or at least one selected from hydrocortisone, prednisolone, dexamethasone and betamethasone, and a molar ratio of glucose / corticosteroid is 10 to $10^2$.

MEANS FOR SOLVING THE PROBLEMS

High Mobility Group Box 1 (HMGB1), which is non-histone protein generally existing in the nucleus of a cell, is involved in generation of a protein complex in a nucleus, preservation of stability of a chromatin structure, control of transcription of various genes and repair of DNA damage. HMGB1 usually exists in a nucleus of a cell, but is released to the outside of the cell when the cell is activated or undergoes necrosis. HMGB1 which is released from the cell activates an inflammatory cell, protects tissue and/or repairs tissue. For example, HMGB1 activates a fibroblast, an epithelium cell, a vascular endothelial cell, a skeletal muscle cell, a cardiac muscle cell, a stem cell and the like, and promotes migration and proliferation thereof.

Having earnestly conducted investigations, the present inventors discovered that glucose can promote production of HMGB1, induce release of HMGB1 to the outside of a cell, increase proliferation and migration of normal cells and promote tissue repair unexpectedly, and achieved the present invention.

Furthermore, the present inventors discovered that glucose can make up disadvantages of a steroid unexpectedly, and achieved the present invention.

Effects of the invention

Due to the present invention, cell damage can be prevented effectively, and repair of various tissues in which damage has been caused can be promoted.

BRIEF DESCRIPTION OF THE DRAWINGS

Fig. 1 is a graph which shows the results of a cell proliferation examination A.
Fig. 2 is a graph which shows the results of a cell proliferation examination B.
Fig. 3 is a graph which shows the results of Example 7 and Comparative Example 5.
Fig. 4 is a graph which shows the results of Examples 8 and 9, Reference Example 1 and Comparative Example 6.

Fig. 5 shows a graph which shows the results of Examples 10 and 11, Reference Example 2 and Comparative Example 7.

BEST MODE FOR CARRYING OUT THE INVENTION

Hereinafter, suitable examples of the present invention are explained, but the present invention is defined by the claims.(Pharmaceutical composition)

A pharmaceutical composition of the present invention includes glucose as a tissue repairing promoter.

Examples of usage of the pharmaceutical composition of the present invention include, for example; agents for treating autoimmune diseases such as atopic dermatitis, bronchial asthma, rheumatoid arthritis and collagen disease; anti-inflammatory agents wherein the examples thereof include agents for treating disease, and examples of the disease include organ inflammation such as nephrotic syndrome, ulcerative colitis, hepatitis, pancreatitis, thyroiditis and cold; and agents for treating a burn, a wound (wound remedy), a bedsore, a pimple, a heat rash and frostbite. The effect of the present invention is particularly useful for anti-inflammatory agents and wound remedies.

The dosage form of the pharmaceutical composition is not particularly limited. Examples of the form include: oral agents such as a tablet, granule, fine granule, powder, a capsule, a pill, liquid drug, emulsion, a suspension, a syrup and a lozenge; percutaneous absorption agents such as an ointment, a lotion, cream and an aerosol agent, and parenteral agents such as an injection, an ophthalmic solution, or a suppository. Among them, remarkable effects of the present invention are shown when the pharmaceutical composition is used as a percutaneous absorption agent such as an ointment.

Here, from the viewpoint of safety, an injection may be prepared such that a liquid agent (pharmaceutical composition) is filled in a vial or the like, water is removed from the agent by freeze-dry treatment, and the freeze-dried agent is dispersed in physiological salt solution or the like immediately before use thereof to obtain a liquid agent.

(Glucose)

A pharmaceutical composition of the present invention includes glucose as a tissue repairing promoter, and therefore, the pharmaceutical composition can promote production of HMGB1 in a cell, induce release of HMGB1 to the outside of the cell and promote tissue repair.

A tissue repair promoter promotes cell proliferation, and improves self-repairing of tissue which includes damaged cell.

The content of glucose included in the pharmaceutical composition can be determined in consideration of a dosage form, usage, administration type and the like of the pharmaceutical composition.

For example, when the composition is a percutaneous absorption agent, 0.1 to 5 mol/L is preferable, 0.1 to 4 mol/L is more preferable, 0.5 to 4 mol/L is still more preferable, and 1 to 3 mol/L is particularly preferable. The aforementioned range may be changed according to conditions, and, for example, 0.05 to 3 mol/L is preferable, and 0.1 to 3 mol/L is also preferable. When the content is equal to or more than the aforementioned lower limit (0.1 mol/L), due to glucose which is absorbed by skin, production and release of HMGB1 is further promoted, cell damage is more suitably prevented, and tissue repair can be sufficiently promoted. When the content is equal to or less than the aforementioned upper limit (5 mol/L), glucose can be dispersed in the pharmaceutical composition uniformly, and cell damage is further suppressed and tissue repair can be further promoted. The content of the glucose included in the pharmaceutical compositions may be changed if necessary in a range of the present invention, and can be determined suitably, and examples thereof include 1 to 90% by mass, 1.5 to 70% by mass, 3 to 50% by mass or 5 to 15% by mass.

When the composition is an injection, it may be used in an amount of 0.5 to 1000 mM/L, preferably 1 to 1000 mM/L, more preferably 1 to 500 mM/L, still more preferably 1 to 300 mM/L, furthermore preferably 1 to 100 mM/L and particularly preferably 10 to 100 mM/L. Furthermore, depending on conditions, 5 to 70 mM/L, 3 to 50 mM/L or 2 to 80 mM/L is also preferable. When the content is within the above range, production and release of HMGB1 are further promoted, cell damage is more preferably prevented and tissue repair can be further promoted.

(Steroid)

The pharmaceutical composition can include a steroid. The pharmaceutical composition can exhibit superior action such as anti-inflammatory action when a steroid is included in the composition. In addition, the effects of the present invention are remarkably exhibited when the pharmaceutical composition includes a steroid. Especially, with respect to the pharmaceutical composition such as an anti-inflammatory agent to which beneficial effect of a steroid such as anti-inflammatory action is required, proliferation inhibiting action provided by a steroid can be remarkably reduced due to glucose.

A steroid usable in the present invention can be determined by taking usage or the like of the pharmaceutical composition into consideration. Examples of the steroid include cortisone acetate, hydrocortisone, hydrocortisone sodium succinate, prednisolone, methylprednisolone, methylprednisolone sodium succinate, triamcinolone, triamcinolone acetonide, dexamethasone, dexamethasone palmitate, betamethasone, paramethasone acetate, fludrocortisone acetate and halopredone acetate. Among these, at least one selected from hydrocortisone, prednisolone, dexamethasone and betamethasone is preferably used from the view point of remarkably exhibiting the effects of the present invention. The steroid may be used singly, or in combination of two or more.

Hydrocortisone is physiological corticosteroid, and has relatively short action time.

Prednisolone is generally used as an oral medicine, has double bonds at the first and the second positions of a steroid skeleton of hydrocortisone and has larger anti-inflammatory action than that of hydrocortisone.

Dexamethasone is synthetic glucocorticoid, wherein fluorine is introduced at the ninth position and a methyl group is introduced at the sixteenth position of prednisolone, and has anti-inflammatory action and antiallergic action which are larger than that of prednisolone. Betamethasone is an isomer of dexamethasone and is known as a strong steroid which acts for a long time.

The content of a steroid included in the pharmaceutical composition can be determined optionally by taking usage of the pharmaceutical composition and the kind of a steroid into consideration. For example, the content may be 0.05 to 0.1% by mass. When the content is within the range, actions of a steroid such as anti-inflammatory action tends to be sufficiently exhibited, and proliferation inhibiting action provided by a steroid can be more satisfactorily reduced due to glucose. The content is not limited to the aforementioned range and may be changed if necessary. As other examples of the content, 0.00001 to 0.1% by mass, 0.00003 to 0.05% by mass, 0.01 to 0.1% by mass, 0.01 to 0.5% by mass, 0.03 to 0.3% by mass and the like can be cited.

A molar ratio represented by "glucose / steroid" in the pharmaceutical composition (hereinafter, it may be referred as "glucose / steroid ratio") can be determined by taking the usage of the pharmaceutical composition and the kind of steroid into consideration. For example, 10 to $10^2$ can be cited. When the ratio is included in the above range, proliferation inhibiting action provided by a steroid can be further suppressed, and tissue repairing tends to be further promoted. In addition, when the ratio is included in the above range, actions of a steroid such as anti-inflammatory action tends to be sufficiently exhibited.

(Optional components)

The pharmaceutical composition can include an optional component (it may be referred especially as a medical optional component) if necessary, in addition to glucose and a steroid. Examples of the medical optional component include pharmacologically acceptable salts, excipients, thickeners, carriers, flavoring agents and dyes. The medical optional component can be used singly, or in combination of two or more.

As the pharmacologically acceptable salts, commonly used salts having no toxicity, that is, acid addition salts and salts obtained using various bases, can be cited. Examples thereof include: inorganic acid salts such as hydrochloride, nitrate and sulfate; organic acid salts such as acetate, citrate, fumarate and tartrate; salts of sulphonic acid such as methane sulfonate and p-toluene sulfonate; amino acid salts such as alanine salt, leucine salt and glutamate; inorganic base salts such alkali metal salts (for example, sodium salt, potassium salt) and alkaline earth metal salts (for example, magnesium salts, calcium salts); and organic amine salts such as triethylamine salts, pyridine salts, picoline salts, ethanolamine salts, triethanolamine salts, dicyclohexylamine salts and N, N'-dibenzyl ethylene diamine salt. The salts may be used singly, or in combination of two or more. When such a salt is included in the composition, crystallization can be easily performed.

An excipient can be suitably selected according to a dosage form of the pharmaceutical composition. Examples thereof include: liquid excipients such as water such as distilled water, demineralized water and pure water, lower alcohols having 1 to 6 carbons such as methanol and ethanol, and glycerin; and solid excipients such as milk sugar, starch, dextrin and saccharose. The excipients can be used singly, or in combination of two or more. For example, the pharmaceutical composition of the present invention may be a pharmaceutical composition which consists of glucose, a steroid and water.

A thickener can be suitably selected based on a dosage form of the pharmaceutical composition. Examples thereof include: gelatine, xanthan gum and carrageenan. The thickeners can be used singly, or in combination of two or more.

Usage of the pharmaceutical composition of the present invention can be determined according to the kinds of diseases or the like.

For example, when the diseases is atopic dermatitis or caused by a wound of skin such as a cut or a scratch, a method or the like can be used wherein the pharmaceutical composition which is a percutaneous absorption agent is directly applied to the wound.

Furthermore, when the affected area is in the mouth, stomach or intestines, a method or the like can be used wherein the pharmaceutical composition is orally administered as an oral agent, or the pharmaceutical composition is injected into the diseased tissue as an injection. Otherwise, in order to promote mucosa regeneration, other methods may be used wherein an affected area is washed by the pharmaceutical composition and/or the pharmaceutical composition is applied to an affected area while the area is observed by the upper endoscopy. Due to such methods, healthy mucosa can be generated, natural healing ability is enhanced and invasion of mucosa can be prevented. Furthermore, when the pharmaceutical composition is coated to gastric mucosa, regeneration of gastric mucosa is promoted, and epithelial tissue can be repaired. By promoting repair of epithelial tissue, that is, by promoting activity of normal cells, growth of abnormal cells such as cancer cells is prevented, and tissue normalization can be induced.

Furthermore, when the diseases is disorder of mucosa part of eye and/or nose such as hay fever, a method or the like can be used wherein the pharmaceutical composition is coated to the affected area. When the pharmaceutical composition is coated to the mucosa part of eye and/or nose, the mucosa part of eye or nose is repaired and a strong barrier is formed at the mucosa layer. Accordingly, even when a lot of allergens are adhered to the mucous part of one's nose and/or eye, an allergic reaction is suppressed, and it is possible to eliminate the occurrence of a stuffy nose and the secretion of an excessive amount of nasal discharge or tears.

In addition, when the diseases is inflammation (influenza) of an upper respiratory mucous membrane which is caused by virus or bacterial infection such as cold, a method or the like can be used wherein the pharmaceutical composition is administered by nasal administration or inhalation. Due to the method, inflammation of the upper respiratory mucous membrane is relieved, the upper respiratory mucous membrane is repaired and regenerated which inhibits infection progress, and activation of natural immune response can be promoted.

A dosage of the pharmaceutical composition can be appropriately determined according to age and weight of a patient, a kind and degree of the diseases, and the administration method. For example, when the pharmaceutical composition includes a steroid, the pharmaceutical composition can be administered such that the composition includes the steroid in an amount which is similar to that of a steroid included in commercially available pharmaceutical compositions. The pharmaceutical composition of the present invention can reduce repair inhibitory action of a steroid with respect to normal cells. Accordingly, the composition may be administered so that the composition includes a steroid in an amount which is larger than the conventionally used amount of the steroid.

(Medical cosmetic)

The medical cosmetic of the present invention includes the pharmaceutical composition of the present invention. Examples of the medical cosmetic of the present invention include: a lotion, a cream, a milky lotion, and a foundation, and the object thereof is to prevent dermatoses. Here, the medical cosmetic is classified as quasi-drugs which are specified by the Japanese Pharmaceutical Affairs Law.

The content of the pharmaceutical composition in the medical cosmetic can be determined in consideration of a dosage form, usage of the medical cosmetic, and the like. For example, it may be used in an amount such that the glucose concentration in the medical cosmetic may be 0.5 to 1000 mM/L, preferably 1 to 1000 mM/L, more preferably 1 to 500 mM/L, still more preferably 1 to 300 mM/L, furthermore preferably 1 to 100 mM/L, and particularly preferably 10 to 100 mM/L. Furthermore, depending on conditions, 5 to 70 mM/L, 3 to 50 mM/L, or 2 to 80 mM/L is preferably used. When the concentration is within the above range, production and release of HMGB1 are further promoted, cell damage is more preferably prevented and tissue repair can be further promoted. Furthermore, when the medical cosmetic including a steroid is involved, the content of the pharmaceutical composition in the medical cosmetic can be determined in consideration of kinds and the like of a steroid.

When the medical cosmetic includes a steroid, a glucose / steroid ratio in the medical cosmetic is preferably 10 to $10^2$. When the ratio is included in the above range, proliferation inhibiting action provided by a steroid can be more reduced, and tissue repairing tends to be further promoted. In addition, when the ratio is included in the above range, actions of a steroid such as anti-inflammatory action tends to be sufficiently exhibited.

The medical cosmetic can include an optional component (it may be referred as a medical cosmetic optional component) other than the pharmaceutical composition if necessary.

Examples of the medical cosmetic optional component include: oils such as liquid paraffin, ceresin, mineral wax, lanoline, vaseline, cetanol, squalene, jojoba oil, stearic acid, palmitic acid, lauryl alcohol, stearyl alcohol, cetyl alchohol, beeswax, methyl polysiloxane and dimethyl cyclopolysiloxane; humectants such as propanol, glycol, propylene glycol, hyaluronic acid, collagen, polyethylene glycol, cholesteryl hydroxystearate, glycerin and sorbitol; various surfactants; emulsifiers; excipients; thickeners; pH regulators; antioxidants; dyes; flavoring agents; and ultraviolet absorbing agents. The medical cosmetic optional component may be used singly, or in combination of two or more.

As described above, the pharmaceutical composition of the present invention includes glucose, and therefore, can effectively prevent cell damage and promote repair of various tissue to which damages have been caused. The mechanism of glucose which prevents cell damage and promotes repair of tissue can be considered as follows. When glucose is administered, IP6K-1 kinase of cytoplasm is activated, and the activated IP6K-1 kinase activates extracellular signal-regulated kinase (ERK). The activated ERK promotes production of HMGB1 in a nucleus, and induces the release of HMGB1 to the outside of the cell. Then, HMGB1 which is released to the outside of the cell promotes migration and proliferation of nonnal cells, activates inflammatory cells, protects tissue, and/or repairs tissue. For example, when the pharmaceutical composition of the present invention is applied on the skin, production of HMGB1 is promoted in skin keratinocyte or skin fibroblasts, and the produced HMGB1 is released to the outside of the cell and promotes a migration and proliferation of skin keratinocyte or epithelial cell, and therefore healing of a wound of skin can be promoted.

In addition, pharmaceutical composition of the present invention can have strong anti-inflammatory action when the pharmaceutical composition includes a steroid, and cell damage can be effectively prevented since proliferation inhibiting action which is provided by a steroid can be reduced due to glucose, and therefore repair of various tissue to which damages have been caused can be prompted.

Furthermore, when a glucose / steroid ratio is included in the specified range, while more highly maintaining actions of a steroid such as anti-inflammatory action, proliferation inhibiting action provided by a steroid is reduced, cell damage is more effectively prevented, and repair of various tissue to which damages have been caused can be further prompted.

The medical cosmetic of the present invention includes the pharmaceutical composition of the present invention, and therefore, can effectively prevent cell damage, and promote repair of various tissue to which damages have been caused.

EXAMPLES

Hereinafter, the present invention is explained in detail using Examples, but the present invention is not limited by the following descriptions.

(Examples 1 to 6 and Comparative Examples 1 to 4)

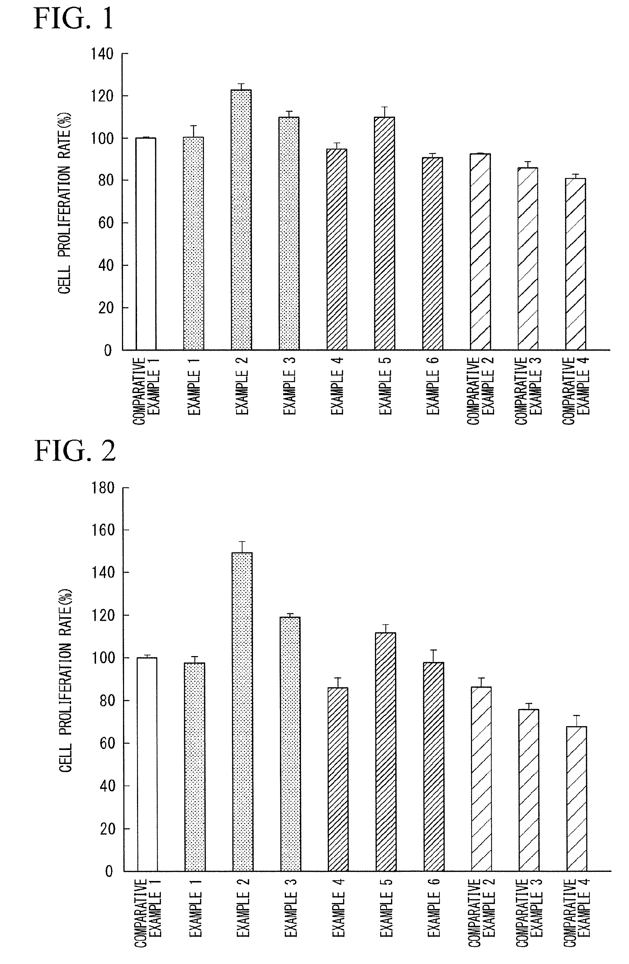

Mediums (hereinafter, it is referred as test mediums) including pharmaceutical compositions were generated such that D-glucose and dexamethasone (product number: D4902, manufactured by Sigma-Aldorich Co.) were added to a Dulbecco's Modified Eagle Medium with 1% by mass of Fetal bovine serum (FBS) (1% FBS-DMEM, manufactured by Life Technologies Corporation) so that the concentrations shown in Table 1 were satisfied. Using each of the test mediums, cell proliferation test shown below was performed. The results thereof are shown in Table 1 and Figs. 1 and 2.

(Cell proliferation test A)

Normal human skin keratinocytes (normal skin keratinocytes derived from single donor, initial culture, manufactured by Takara Bio Inc.) were seeded to a 96-well microplate using 1% FBS-DMEM so that 20000 cells/well were provided, and culture was performed for 24 hours at a temperature of 37°C (pre-culture). Subsequent to the pre-culture, the medium used in the pre-culture was replaced with the aforementioned test mediums of each example, and culture was performed for 24 hours at a temperature of 37°C (main-culture). After the main culture, 1% FBS-DMEM which includes 0.4 mg/mL of 3-(4,5-dimethy1-2-thiazolyl)-2,5-diphenyltetrazolium bromide (MTT) was replaced with the test medium, and culture was performed for 4 hours at a temperature of 37°C (post-culture). After the post-culture, the medium was removed, and dimethyl sulfoxide (100 pt/well) was added and mixed to prepare a sample solution. Absorbance (550 nm) of the sample solution was measured to obtain a reduction amount of MTT (a). Furthermore, sample solution (control sample solution) was prepared similar to the aforementioned method except that the main culture was not performed, and adsorption (550 nm) of the control sample solution was measured to obtain a reduction amount of MTT ($\beta$). A cell proliferation rate was obtained according to the formula (1) shown below.

$$\text{Cell proliferation rate (\%)} = \text{a reduction amount of MTT (a) / a reduction amount of MTT}(\beta) \times 100 \dots(1)$$

(Cell proliferation test B)

Similar to the cell proliferation test A, a cell proliferation rate was obtained except that normal human skin fibroblasts (normal skin fibroblasts derived from single donor, initial culture, manufactured by Takara Bio Inc.) was used instead of the normal human skin keratinocytes.

Table 1

| | | Examples | | | | | | Comparative Examples | | | |
|---|---|---|---|---|---|---|---|---|---|---|---|
| | | 1 | 2 | 3 | 4 | 5 | 6 | 1 | 2 | 3 | 4 |
| Concentration | D-glucose (mM/L) | 1 | 10 | 100 | 1 | 10 | 100 | - | - | - | - |
| | Dexamethasone (μM/L) | - | - | - | 100 | 100 | 100 | - | 10 | 100 | 1000 |
| | Glucose / Steroid ratio | - | - | - | 10 | $10^2$ | $10^3$ | - | - | - | - |
| Results | Cell proliferation test A (%) | 101 | 123 | 110 | 95 | 110 | 91 | 100 | 93 | 86 | 81 |
| | Cell proliferation test B (%) | 98 | 150 | 120 | 86 | 112 | 98 | 100 | 87 | 76 | 68 |

Fig. 1 is a graph which shows the results of the cell proliferation examination A, and Fig. 2 is a graph which shows the results of the celle proliferation examination B. As shown in Table 1 and Figs. 1 and 2, the results of Examples 1 to 3 to which the present invention was applied shown that the cell prolifeartion rates thereof were nearly the same as 100% or exceeded 100%. Particularly, an increase in the cell proliferation rate of Examples 2 and 3 wherein 10 to 100 mM/L of glucose was included was remarkable.

On the other hand, with respect to Comparative Example 1 wherein neither glucose nor dexamethasone was included, the cell proliferation rates obtained in the cell proliferation examinations A and B were 100%.

As the results of Examples 1 to 3 and Comparative Example 1, it was confirmed that the growth of cells can be promoted due to the addition of glucose.

As shown in the results of Comparative Examples 2 to 4, the cell proliferation rates decreased as the amount of dexamethasone increased. By the results, it was confirmed that proliferation of normal cells was inhibited due to dexamethasone.

In the cell proliferation tests A and B, Examples 4 to 6 wherein glucose and dexamethasone were included showed higher cell proliferation rates as compared with Comparative Example 3 wherein dexamethasone is included in an amount similar to those of Examples 4 to 6 but no glucose was included. From the results of Examples 4 to 6 and Comparative Example 3, it was confirmed that the proliferation inhibiting effect with respect to normal cells, which was caused by dexamethasone, was reduced by glucose.

As the results described above, it was confirmed that, due to the application of the present invention, the proliferation of normal cells can be promoted, cell damage can be prevented and repair of various tissues to which damage has been caused can be promoted. The proper amount of glucose can be determined appropriately according to usage in the scope of the present invention.

(Example 7)

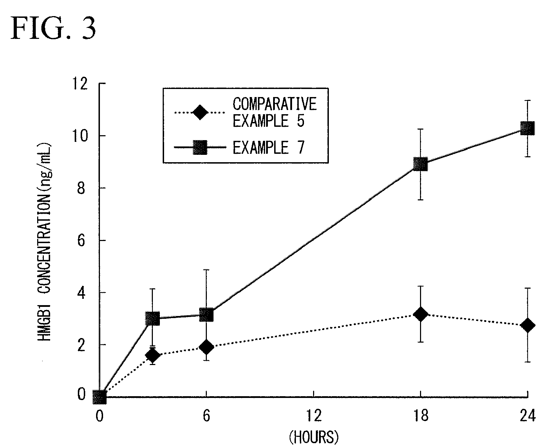

Glucose and normal human skin keratinocytes were added to 1% FBS-DMEM to prepare a suspension sample wherein the glucose concentration was 10 mM/L and the content of the normal human skin keratinocytes was 100000 cells/mL. Culture of 100 μL of the suspension sample was performed for 24 hours in a $CO_2$ incubator (37°C). The supernatants of the sample were collected at the timing of: before the start of culture (0 hours), 3 hours after the start, 6 hours after the start, 18 hours after the start and 24 hours after the start, and the concentration of HMGB1 in the collected supernatants was measured by HMGB1 ELISA Kit II (manufactured by Shino-Test Corporation). The results were shown in Fig. 3.

(Comparative Example 5)

The concentration of HMGB1 in supernatants was measured similar to Example 7, except that a suspension sample wherein glucose was not included was used. The results were shown in Fig. 3.

As shown in Fig. 3, Example 7 wherein glucose was added has a higher HMGB1 concentration as compared with that of Comparative Example 5. By the results, it was confirmed that glucose promotes the release of HMGB1 from normal human skin keratinocytes.

(Example 8)

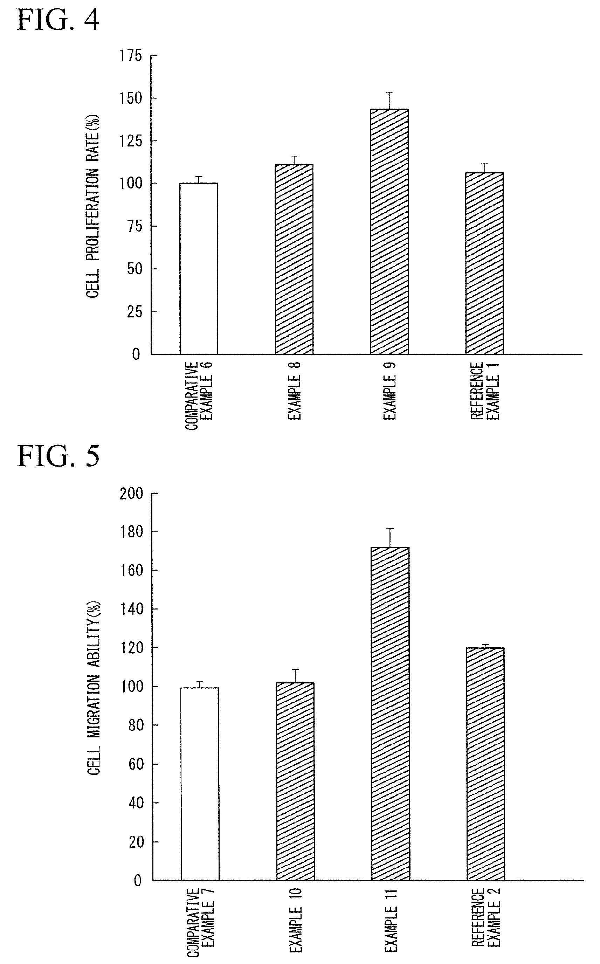

1% FBS-DMEM wherein the glucose concentration thereof was 10 mM/L was prepared, and was used as a test medium. A cell proliferation rate was obtained similar to that of the cell proliferation test A, except that pre-culture was not performed and 10000 cells/well of normal human skin keratinocytes were seeded to a microplate. The results thereof were shown in Fig. 4.

(Example 9)

Glucose and normal human skin fibroblasts were added to 1% FBS-DMEM to prepare a suspension sample, wherein the glucose concentration thereof was 10 mM/L and the content of the normal human skin fibroblasts was 100000 cells/mL. Culture of the suspension sample was performed for 24 hours at 37°C, and a supernatant (culturing supernatant) thereof was collected. 100 μL of the culturing suspension was added to 10000 cells of normal human skin keratinocytes, and cultured for 24 hours at 37°C. After culturing, a cell proliferation rate was obtained similar to Example 8. The results thereof were shown in Fig. 4.

(Reference Example 1)

A cell proliferation rate was obtained similar to Example 9, except that anti-HMGB1 chicken polyclonal antibody (IgY fraction) was added in an amount of 10 pg/mL to a test medium. The results thereof were shown in Fig. 4.

(Comparative Example 6)

A cell proliferation rate was obtained similar to Example 8 except that glucose was not added. The results thereof were shown in Fig. 4.
As shown in Fig. 4, the cell proliferation rate of Example 8 wherein glucose was added to the test medium was 110%. Furthermore, the cell proliferation rate of Example 9 to which the culturing supernatant was added was 142%. That is, due to the addition of the culturing supernatant, proliferation of the nomial human skin keratinocytes was further promoted.
On the other hand, the cell proliferation rate of Comparative Example 6 to which neither glucose nor culturing supernatant was added was 100%.
Furthermore, the cell proliferation rate of Reference Example 1 wherein anti-HMGB1 antibody was added was 106%, and the cell proliferation rate was lower than that of Example 9. That is, proliferation of the normal human skin keratinocytes was inhibited when HMGB1 was inactivated. From the results of Examples 8 and 9 and Reference Example 1, it was confirmed that the culturing supernatants included HMGB1 which was released due to the presence of glucose, and the proliferation of the normal human skin keratinocytes was promoted by the HMGB1.
As the results of Figs. 3 and 4, it was confirmed that glucose induces the release of HMGB1 from the normal human skin fibroblasts, and the released HMGB1 promotes the proliferation of the normal human skin keratinocytes.
Accordingly, it was confirmed that, due to the application of the present invention, it is possible to promote proliferation of normal cells and promote the repair of tissues.

(Example 10)

1% FBS-DMEM wherein the glucose concentration was 10 mM/L was prepared as a test medium, and the test medium was added to a lower layer of a cell migration chamber (manufactured by Becton, Dickinson and Company). 100000 cells of normal human skin keratinocytes were seeded to the insert equipped with a filter (pore size thereof is 8μm, manufactured by Becton, Dickinson and Company). The insert to which seeding was performed was provided on a 24-well plate, and culture was performed for two hours under an atmosphere of 5% by volume $CO_2$ at 37°C. The cells of the upper layer of the filter were scraped with a cotton swab, the cells of the lower layer were fixed by methanol, and hematoxylin staining was performed. An average numbers of the cell observed in five views (magnification of 200), which were randomly selected, was determined as a number of migrated cells of Example 11. A number of migrated cells, which was observed in Comparative Example 7 described below, was set to a reference number of migrated cell, and the cell migration ability was obtained by the following formula (2). The results were shown in Fig. 5.

Cell migration ability (%) = a number of migrated cells of each Example / a reference number of migrated cells x 100 … (2)

(Example 11)

Glucose and normal human skin fibroblasts were added to 1% FBS-DMEM to prepare a suspension, wherein the glucose concentration was 10 mM/L and the content of the normal human skin fibroblasts was 100000 cells/mL. Culture of the suspension was performed for 24 hours at 37°C, and a supernatant (culturing supernatant) thereof was collected. Cell migration ability was obtained similar to Example 10, except that a test medium was prepared by mixing 500 μL of the culturing suspension and 500 μL of 1% FBS-DMEM having 10 mM/ L of glucose concentration. The results are shown in Fig. 5.

(Reference Example 2)

Cell migration ability was obtained similar to Example 11, except that anti-HMGB1 antibody was furthermore added to the test medium so that the content of the anti-HMGB1 antibody is10 μg/mL The results are shown in Fig. 5.

(Comparative Example 7)

Cell migration ability was obtained similar to Example 10, except that glucose was not added. The results are shown in Fig. 5.

As shown in Fig. 5, the cell migration ability of Example 10 wherein glucose was added to the test medium was 102%. The cell migration ability of Example 11, wherein the culturing supernatant of the suspension sample to which glucose was furthermore added, was 171%. That is, due to the addition of the culturing supernatant, migration of the normal human skin keratinocytes was induced.

On the other hand, the cell migration ability of Comparative Example 7 to which neither glucose nor culturing supernatant was added was 100%.

Furthermore, the cell migration ability of Reference Example 2 wherein anti-HMGB1 antibody was added was 116%, and the cell migration ability was lower than that of Example 11. That is, migration of the normal human skin keratinocytes was further promoted, when HMGB1 was inactivated. From the results of Examples 10 and 11 and Reference Example 2, it was confirmed that HMGB1 which was released due to the presence of glucose was included in the culturing supernatants, and the migration of the normal human skin keratinocytes was promoted by the HMGB1.

As the results of Figs. 3 and 5, it was confirmed that glucose induces the release of HMGB1 from the normal human skin fibroblasts, and the released HMGB1 promotes the migration of the normal human skin keratinocytes.

Furthermore, it was confirmed that, due to the application of the present invention, it is possible to promote migration of normal cell and promote repair of tissues.

(Example 12)

An aqueous solution containing 1.9 mol/L of glucose and 0.096 mol/L of a steroid (dexamethasone sodium phosphate) was prepared, and was used as an external medicine.

The external medicine was applied once a day to an affected part of a patient (male in his twenties) who had skin itchiness, pigmentation and lichenization on the whole skin (face, upper body, lower body and fingers and toes) thereof. As a result of the application, his skin did not feel itchiness the day after application was started, and pigmentation and lichenization were eliminated three months after application was started. Before application of the external medicine was started, the concentration of IgE in serum of the patient was 5500, and thymus and activation-regulated chemokine (TARC) of the patient was 5300. Three month after the application of the external medicine was started, the concentration of IgE in serum of the patient was 2700 to 2800, and TARC fell to 170, and therefore remarkable effects were confirmed with respect to allergic reaction. Both the concentration of IgE in serum and TARC are indexes which show seriousness of an allergic disease.

## Claims

1. A composition which comprises glucose and a corticosteroid for use in promoting tissue repair in a subject in need thereof, wherein

   the corticosteroid is dexamethasone or dexamethasone sodium phosphate, or at least one selected from hydrocortisone, prednisolone, dexamethasone and betamethasone, and
   the molar ratio of glucose / corticosteroid is 10 to $10^2$.

2. The composition for use according to Claim 1, wherein the composition is part of a cosmetic.

3. The composition for use according to Claim 1, which composition is an anti-inflammatory agent, or an agent for use in treating autoimmune diseases or treating a wound.

**4.** The composition for use according to Claim 1, which composition is an oral agent, a percutaneous absorption agent or a parenteral agent.

**5.** The composition for use according to Claim 1, which composition is an ointment.

**6.** The composition for use according to Claim 1, wherein the composition is a percutaneous absorption agent, and the content of glucose in the composition is 0.1 to 5 mol/L.

**7.** The composition for use according to Claim 1, wherein

the composition is a percutaneous absorption agent, and
the content of glucose in the composition is 1 to 3 mol/L.

**8.** The composition for use according to Claim 1, wherein

the composition is in an injectable form, and
the content of glucose included in the composition is 1 to 1000 mM/L.

**9.** The composition for use according to Claim 1, wherein

the composition is in an injectable form, and
the content of glucose included in the composition is 10 to 100 mM/L.

**10.** The composition for use according to Claim 1, wherein

the composition comprises a corticosteroid,
the steroid is at least one selected from the group consisting of cortisone acetate, hydrocortisone, hydrocortisone sodium succinate, prednisolone, methylprednisolone, methylprednisolone sodium succinate, triamcinolone, triamcinolone acetonide, dexamethasone, dexamethasone palmitate, betamethasone, paramethasone acetate, fludrocortisone acetate and halopredone acetate;
the corticosteroid is included in an amount of 0.05 to 0.1 % by mass.

**11.** The composition for use according to Claim 1, wherein

the composition consists of glucose, a corticosteroid and water;
the content of glucose in the composition is 0.1 to 5 mol/L.

**12.** The composition for use according to Claim 1, wherein

the composition is a cell migration promoter.


**Patentansprüche**

**1.** Zusammensetzung, die Glucose und ein Corticosteroid umfasst, zum Verwenden beim Fördern von Gewebereparatur in einem Subjekt, das dies benötigt, wobei
das Corticosteroid Dexamethason oder Dexamethason-Natriumphosphat oder wenigstens eines ausgewählt aus Hydrocortison, Prednisolon, Dexamethason und Betamethason ist und
das Molverhältnis von Glucose zu Corticosteroid 10 zu $10^2$ beträgt.

**2.** Zusammensetzung zum Verwenden nach Anspruch 1, wobei die Zusammensetzung Teil eines Kosmetikums ist.

**3.** Zusammensetzung zum Verwenden nach Anspruch 1, wobei die Zusammensetzung ein entzündungshemmendes Mittel oder ein Mittel zum Verwenden beim Behandeln von Autoimmunerkrankungen oder beim Behandeln einer Wunde ist.

**4.** Zusammensetzung zum Verwenden nach Anspruch 1, wobei die Zusammensetzung ein orales Mittel, ein Mittel zum perkutanen Absorbieren oder ein parenterales Mittel ist.

**5.** Zusammensetzung zum Verwenden nach Anspruch 1, wobei die Zusammensetzung eine Salbe ist.

**6.** Zusammensetzung zum Verwenden nach Anspruch 1, wobei die Zusammensetzung ein Mittel zum perkutanen Absorbieren ist und der Glucosegehalt in der Zusammensetzung 0,1 bis 5 mol/l beträgt.

**7.** Zusammensetzung zum Verwenden nach Anspruch 1, wobei die Zusammensetzung ein Mittel zum perkutanen Absorbieren ist und der Glucosegehalt in der Zusammensetzung 1 bis 3 mol/l beträgt.

**8.** Zusammensetzung zum Verwenden nach Anspruch 1, wobei die Zusammensetzung in einer injizierbaren Form vorliegt und der in der Zusammensetzung enthaltene Glucosegehalt 1 bis 1000 mM/l beträgt.

**9.** Zusammensetzung zum Verwenden nach Anspruch 1, wobei die Zusammensetzung in einer injizierbaren Form vorliegt und der in der Zusammensetzung enthaltene Glucosegehalt 10 bis 100 mM/l beträgt.

**10.** Zusammensetzung zum Verwenden nach Anspruch 1, wobei die Zusammensetzung ein Corticosteroid umfasst, das Steroid wenigstens eines ist, ausgewählt aus der Gruppe bestehend aus Cortisonacetat, Hydrocortison, Hydrocortison-Natriumsuccinat, Prednisolon, Methylprednisolon, Methylprednisolon-Natriumsuccinat, Triamcinolon, Triamcinolon-Acetonid, Dexamethason, Dexamethason-Palmitat, Betamethason, Paramethason-Acetat, Fludrocortison-Acetat und Halopredon-Acetat; das Corticosteroid in einer Menge von 0,05 bis 0,1 Gew.-% enthalten ist.

**11.** Zusammensetzung zum Verwenden nach Anspruch 1, wobei die Zusammensetzung aus Glucose, einem Corticosteroid und Wasser besteht; der Glucosegehalt in der Zusammensetzung 0,1 bis 5 mol/l beträgt.

**12.** Zusammensetzung zum Verwenden nach Anspruch 1, wobei die Zusammensetzung ein Zellmigrationspromotor ist.

**Revendications**

**1.** Composition comprenant du glucose et un corticostéroïde pour une utilisation dans la promotion de la réparation des tissus chez un patient en ayant besoin, dans laquelle le corticostéroïde est la dexaméthasone ou le phosphate de sodium de dexaméthasone, ou au moins une choisie parmi l'hydrocortisone, la prednisolone, la dexaméthasone et la bétaméthasone, et le rapport molaire glucose/corticostéroïde est compris entre 10 et $10^2$.

**2.** Composition pour une utilisation selon la revendication 1, dans laquelle la composition fait partie d'un produit cosmétique.

**3.** Composition pour une utilisation selon la revendication 1, laquelle composition est un agent anti-inflammatoire ou un agent pour une utilisation dans le traitement de maladies auto-immunes ou dans le traitement d'une plaie.

**4.** Composition pour une utilisation selon la revendication 1, laquelle composition est un agent oral, un agent favorisant l'absorption percutanée ou un agent parentéral.

**5.** Composition pour une utilisation selon la revendication 1, laquelle composition est un onguent.

**6.** Composition pour une utilisation selon la revendication 1, dans laquelle la composition est un agent favorisant l'absorption percutanée et dans laquelle la teneur en glucose de la composition est comprise entre 0,1 et 5 mol/l.

**7.** Composition pour une utilisation selon la revendication 1, dans laquelle la composition est un agent favorisant l'absorption percutanée, et la teneur en glucose de la composition est comprise entre 1 et 3 mol/l.

**8.** Composition pour une utilisation selon la revendication 1, dans laquelle
la composition est sous une forme injectable, et
la teneur en glucose de la composition est comprise entre 1 et 1 000 mM/l.

**9.** Composition pour une utilisation selon la revendication 1, dans laquelle
la composition est sous une forme injectable, et
la teneur en glucose de la composition est comprise entre 10 et 100 mM/l.

**10.** Composition pour une utilisation selon la revendication 1, dans laquelle
la composition comprend un corticostéroïde,
le stéroïde est au moins un choisi dans le groupe constitué par l'acétate de cortisone, l'hydrocortisone, le succinate de sodium d'hydrocortisone, la prednisolone, la méthylprednisolone, le succinate de sodium de méthylprednisolone, la triamcinolone, l'acétonide de triamcinolone, la dexaméthasone, le palmitate de dexaméthasone, la bétaméthasone, l'acétate de paraméthasone, l'acétate de fludrocortisone et l'acétate d'haloprédone ;
le corticostéroïde est inclus dans une quantité comprise entre 0,05 et 0,1 % en masse.

**11.** Composition pour une utilisation selon la revendication 1, dans laquelle
la composition est composée de glucose, d'un corticostéroïde et d'eau ;
la teneur en glucose de la composition est comprise entre 0,1 et 5 mol/l.

**12.** Composition pour une utilisation selon la revendication 1, dans laquelle la composition est un promoteur de migration cellulaire.

# FIG. 1

# FIG. 2

# FIG. 3

FIG. 4

FIG. 5

**EP 2 808 025 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012011342 A
- JP 2008273919 A
- JP 2007182384 A
- US 5885978 A
- US 20050175640 A

**Non-patent literature cited in the description**

- **SPRAVCHIKOV N et al.** GLUCOSE EFFECTS ON SKIN KERATINOCYTES IMPLICATIONS FOR DIABETES SKIN COMPLICATIONS. *Diabetes,* 2001, vol. 50, 1627-1635